(19) **Europäisches Patentamt / European Patent Office / Office européen des brevets**

(11) **EP 4 440 680 B1**

(12) **EUROPEAN PATENT SPECIFICATION**

(45) Date of publication and mention
of the grant of the patent:
**12.11.2025 Bulletin 2025/46**

(21) Application number: **22826299.4**

(22) Date of filing: **29.11.2022**

(51) International Patent Classification (IPC):
***A61M 60/178*** (2021.01)   ***A61M 60/216*** (2021.01)
***A61M 60/873*** (2021.01)

(52) Cooperative Patent Classification (CPC):
**A61M 60/178; A61M 60/216; A61M 60/873;**
A61M 2205/8243; H02J 50/12; H02J 2310/23

(86) International application number:
**PCT/US2022/080539**

(87) International publication number:
**WO 2023/102365 (08.06.2023 Gazette 2023/23)**

(54) **SYSTEMS AND METHODS FOR WIRELESS POWER RESONATORS WITH OPEN CORE**

SYSTEME UND VERFAHREN FÜR DRAHTLOSE STROMRESONATOREN MIT OFFENEM KERN

SYSTÈMES ET PROCÉDÉS POUR RÉSONATEURS DE PUISSANCE SANS FIL À NOYAU OUVERT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:   **30.11.2021   US 202163284278 P**

(43) Date of publication of application:
**09.10.2024 Bulletin 2024/41**

(73) Proprietor: **TC1 LLC**
**St. Paul, MN 55117 (US)**

(72) Inventors:
• **HANSEN, John Freddy**
**St. Paul, Minnesota 55117 (US)**
• **KEEN, Rachel**
**St. Paul, Minnesota 55117 (US)**
• **HARJES, Daniel I.**
**St. Paul, Minnesota 55117 (US)**
• **ANDERSON, Russell Eugene**
**St. Paul, Minnesota 55117 (US)**

(74) Representative: **Deambrogi, Edgardo et al**
**Jacobacci & Partners S.p.A.**
**Corso Emilia 8**
**10152 Torino (IT)**

(56) References cited:
**WO-A1-2021/252429      US-A1- 2017 259 677
US-A1- 2021 271 790**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### CROSS-REFERENCE TO RELATED APPLICATIONS

This application claims priority to provisional application serial No. 63/284,278, filed November 30, 2021.

### BACKGROUND OF THE DISCLOSURE

#### a. Field of the Disclosure

The present disclosure relates generally to wireless power transfer systems, and more specifically, relates to wireless power transfer resonators including an open core.

#### b. Background

Ventricular assist devices, known as VADs, are implantable blood pumps used for both short-term (i.e., days or months) and long-term (i.e., years or a lifetime) applications where a patient's heart is incapable of providing adequate circulation, commonly referred to as heart failure or congestive heart failure. A patient suffering from heart failure may use a VAD while awaiting a heart transplant or as a long-term destination therapy. In another example, a patient may use a VAD while recovering from heart surgery. Thus, a VAD can supplement a weak heart (i.e., partial support) or can effectively replace the natural heart's function.

A wireless power transfer system may be used to supply power to the VAD. The wireless power transfer system generally includes an external transmit resonator and an implantable receive resonator configured to be implanted inside a patient's body. This power transfer system may be referred to as a transcutaneous energy transfer system (TETS). US 2021/271790 discloses a wireless power transfer system including a transmit resonator and a receive resonator, each of the transmit resonator and the receive resonator including a magnetic core having a post, and a plurality of alternating dielectric layers and conductive layers stacked around the post.

In general, it is desirable to reduce far-field electromagnetic (EM) emissions from a TETS (e.g., to reduce EM interference with other devices). The transmit resonator in a TETS is generally the largest source of far-field EM emissions. Accordingly, it would be advantageous to reduce EM emissions by the transmit resonator and to improve cooling of the transmit resonator.

### SUMMARY OF THE DISCLOSURE

The present disclosure is directed to a resonator for use in a wireless power transfer system. The invention is defined by the features of independent claims 1, 8, and 11.

In one aspect, a resonator for use in a transcutaneous energy transfer system (TETS) is provided. The resonator includes a housing, a magnetic core positioned within the housing, the magnetic core defining an annular groove and a central aperture, a coil element positioned within the annular groove, and at least one layer positioned within the central aperture, the at least one layer comprising a non-magnetic, non-metallic material.

In another aspect, a wireless power transfer system is provided. The wireless power transfer system includes an implantable receive resonator, and an external transmit resonator including a housing, a magnetic core positioned within the housing, the magnetic core defining an annular groove and a central aperture, a coil element positioned within the annular groove, and at least one layer positioned within the central aperture, the at least one layer comprising a non-magnetic, non-metallic material.

In yet another aspect, a method of assembling a resonator for use in a transcutaneous energy transfer system (TETS) is provided. The method includes positioning a magnetic core within a housing, the magnetic core defining an annular groove and a central aperture, positioning a coil element positioned within the annular groove, and positioning at least one layer within the central aperture, the at least one layer comprising a non-magnetic, non-metallic material.

### BRIEF DESCRIPTION OF THE DRAWINGS

FIG. 1 is a simplified electrical circuit diagram of a wireless power transfer system according to an example embodiment.

FIG. 2 is an illustration of the wireless power transfer system of FIG. 1 used to supply power to a ventricular assist device (VAD) according to an example embodiment.

FIG. 3 is a front perspective view of one example of a resonator that may be used to implement the system shown in FIG. 1 according to an example embodiment.

FIG. 4 is a perspective, cross-sectional view of a resonator assembly that may be used to implement the system shown in FIG. 1 according to an example embodiment.

FIG. 5 is cross-sectional view of an alternative resonator that may be used to implement the system shown in FIG. 1.

FIG. 6 is a diagram illustrating magnetic field lines of the resonator shown in FIG. 5.

## DETAILED DESCRIPTION OF THE DISCLOSURE

The present disclosure is directed to systems and methods for wireless power transfer. A resonator includes a housing, a magnetic core positioned within the housing, the magnetic core defining an annular groove and a central aperture, a coil element positioned within the annular groove, and at least one layer positioned within the central aperture, the at least one layer comprising a non-magnetic, non-metallic material.

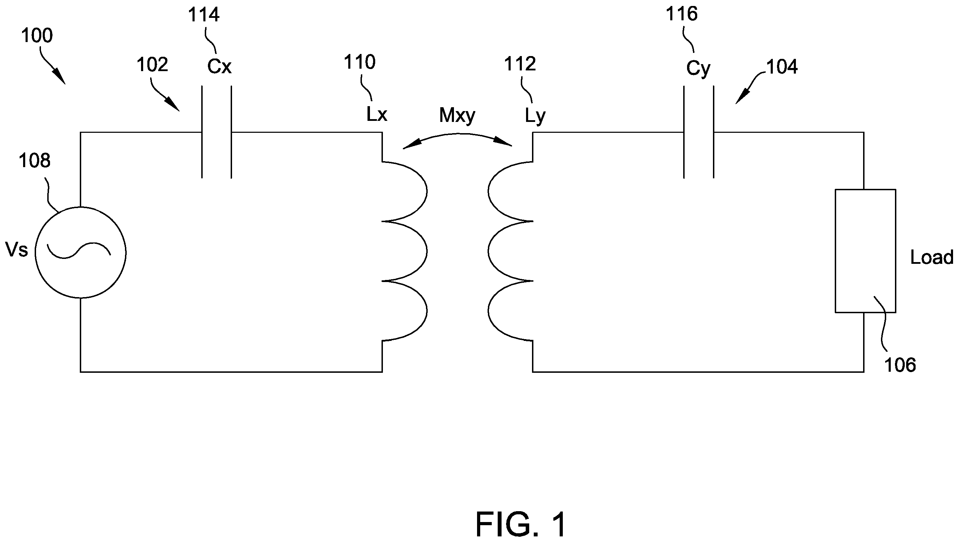

Referring now to the drawings, FIG. 1 illustrates a simplified circuit of a wireless power transfer system 100 according to an example embodiment. The system 100 includes an external transmit resonator 102 and an implantable receive resonator 104. In the system shown in FIG. 1, a power source Vs 108 is electrically connected with the transmit resonator 102, thereby providing power to the transmit resonator 102. The receive resonator 104 is connected to a load 106 (e.g., an implantable medical device). The receive resonator 104 and the load 106 may be electrically connected with a switching or rectifying device (not shown).

In an example, the transmit resonator 102 includes a coil Lx 110 connected to the power source Vs 108 by a capacitor Cx 114. Further, the receive resonator 104 includes a coil Ly 112 connected to the load 106 by a capacitor Cy 116. Inductors Lx 110 and Ly 112 are coupled by a coupling coefficient k. $M_{xy}$ is the mutual inductance between the two coils. The mutual inductance, $M_{xy}$, is related to the coupling coefficient k as shown in the below Equation (1).

$$M_{xy} = k\sqrt{L_x \cdot L_y} \tag{1}$$

In operation, the transmit resonator 102 transmits wireless power received from the power source Vs 108. Receive resonator 104 receives the power wirelessly transmitted by transmit resonator 102 and transmits the received power to load 106.

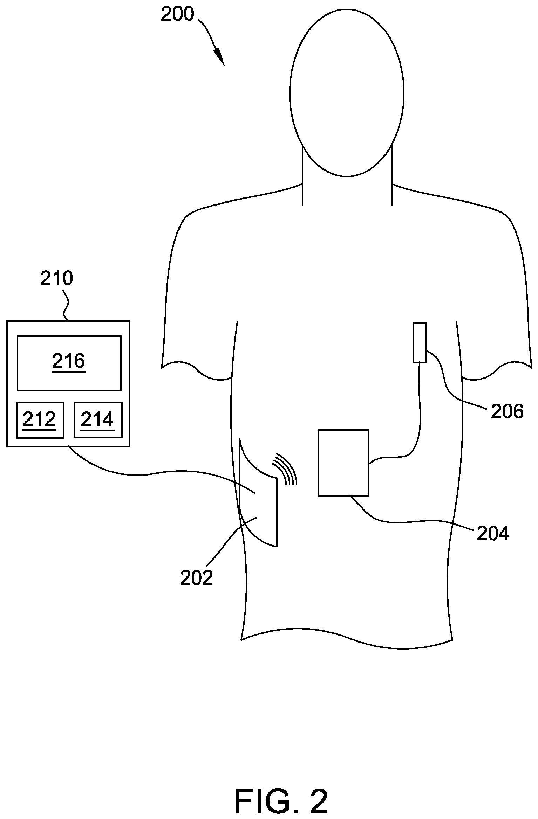

FIG. 2 illustrates an example of a patient 200 using an external coil 202 (e.g., a transmit resonator 102 (FIG. 1)) to wirelessly transmit power to an implanted coil 204 (e.g., a receive resonator 104 (FIG. 1)). Implanted coil 204 uses the received power to power an implanted device 206. For example, implanted device 206 may include a pacemaker or heart pump (e.g., a left ventricular assist device (LVAD)). In some examples, implanted coil 204 and/or implanted device 206 may include or be coupled to a battery.

In one example, external coil 202 is communicatively coupled to a computing device 210, for example, via wired or wireless connection, such that the external coil 202 may receive signals from and transmit signals to the computing device 210. In some examples, the computing device 210 is a power source for the external coil 202. In other examples, the external coil 202 is coupled to an alternative power supply (not shown). The computing device 210 includes a processor 212 in communication with a memory 214. In some examples, executable instructions are stored in the memory 214.

The computing device 210 further includes a user interface (UI) 216. The UI 216 presents information to a user (e.g., the patient 200). For example, the UI 216 may include a display adapter that may be coupled to a display device, such as a cathode ray tube (CRT), a liquid crystal display (LCD), an organic LED (OLED) display, and/or an electronic ink display. In some examples, the UI 216 includes one or more display devices. Further, in some examples, the UI may be or otherwise include a presentation interface. The presentation interface may not generate visual content, but may generate audible and/or computer-generated spoken-word content. In an example, the UI 216 displays one or more representations designed to aid the patient 200 in placing the external coil 202 such that the coupling between the external coil 202 and the implanted coil 204 is optimal. In some examples, the computing device 210 may be a wearable device such as, for example, a wristwatch.

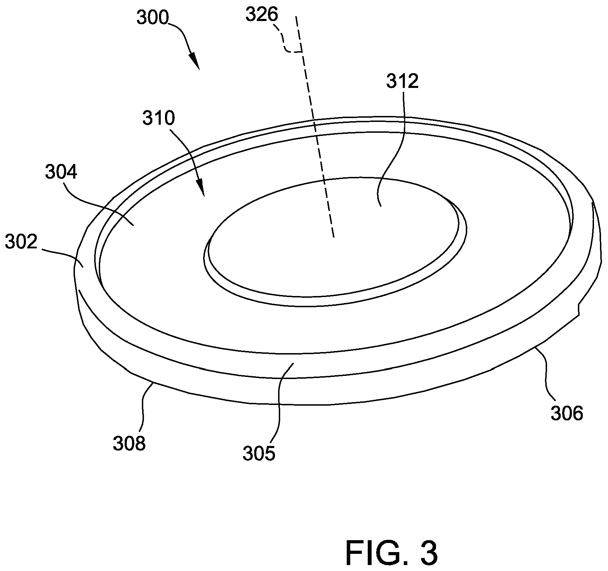

FIG. 3 is a front perspective view of one example of a resonator 300 that may be used to implement the system 100 shown in FIG. 1. For example, the resonator 300 may be used to implement the external transmit resonator 102 (FIG. 1), the implantable receive resonator 104 (FIG. 1), the external coil 202 (FIG. 2), and/or the implanted coil 204 (FIG. 2).

In an example, the resonator 300 includes a core 302 and a coil element 304. The core 302 includes a front surface 305, a back surface 306, and an annular sidewall 308 extending between the front surface 305 and the back surface 306. An annular groove 310 is defined by the front surface 305 and forms a central post 312 of the core 302.

The resonator 300 (including the core 302 and the coil element 304) functions as a wireless power resonator when coupled to a capacitor (e.g., a capacitor on a printed circuit board electrically coupled to coil element 304). However, those of skill in the art will appreciate that resonator 300, without connection to a capacitor, constitutes a coil assembly. Accordingly, as used herein, the term resonator does not require that the device be coupled to a capacitor to form a wireless power resonator. In contrast, as used herein, the term resonator is broad enough to cover a coil assembly that includes a core and a coil element without connection to a capacitor, as shown in FIG 3.

In an example, the core 302 is formed of a magnetic material. The magnetic material may be a ferrite material, such as nickel-based or manganese-based ferrites. Nickel-based ferrites generally have lower electrical conductivity and reduced losses, while manganese-based ferrites have a higher magnetic permeability (while still having acceptable losses), facilitating containing magnetic field lines, and reducing fringing fields entering nearby conductors (e.g., a titanium enclosure or copper in a nearby PCB) to prevent losses. In other examples, other types of ferrite materials may be used. For example, in some examples, a magnesium-based ferrite (e.g., MgCuZn, which may outperform nickel-based and manganese-based ferrites in a frequency range around 1 Megahertz (MHz)) may be used.

The coil element 304 is positioned within the annular groove 310 and surrounds the central post 312. The resonator 300 may be, for example, a Litz wire resonator or a stacked plate resonator. In a Litz wire resonator, the coil element 304 includes a plurality of loops of Litz wire. In a stacked plate resonator, the coil element 304 includes a plurality of stacked plates that may include a plurality of alternating dielectric layers and conductive layers arranged in a stack. The dielectric layers may be formed of, for example, ceramic, plastic, glass, and/or mica.

The coil element 304 may be electrically coupled to a power source (e.g., when functioning as a transmit resonator) or a load (e.g., when functioning as a receive resonator). In operation, when power is supplied to the resonator 300 operating as a transmit resonator, current flows through the coil element 304, creating an inductive current loop. This inductive current loop is capable of wirelessly transmitting power to a second resonator 300, provided that resonance frequencies of the first and second resonators 300 overlap. The coil element 304 may include a plurality of terminals (not shown) that facilitate electrically coupling the coil element 304 to a power supply or load.

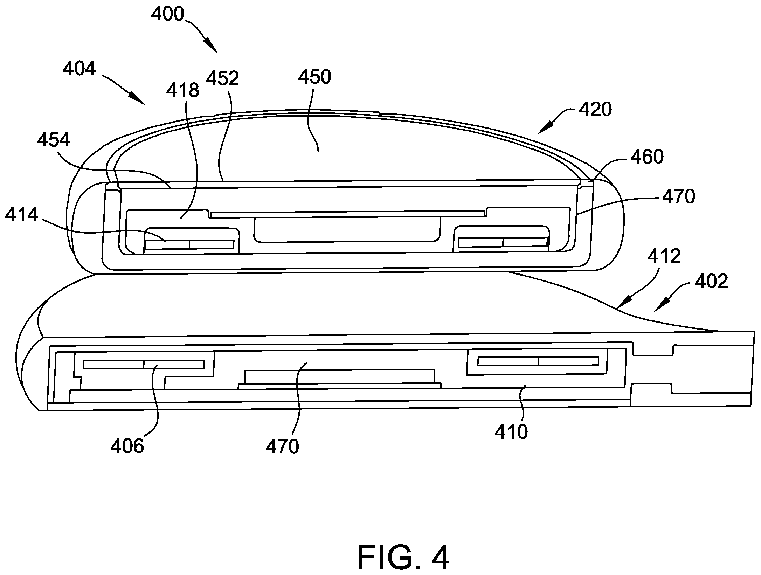

FIG. 4 is a perspective view of a resonator assembly 400. Resonator assembly 400 may include resonators similar to the resonator 300 shown in FIG. 3. The resonator assembly 400 includes a transmit resonator 402 and a receive resonator 404. In an example, the transmit resonator 402 includes a first coil element 406 and a first core 410 positioned within a first housing 412 (e.g., a ceramic housing). Similarly, the receive resonator 404 includes a second coil element 414 and a second core 418 positioned within a second housing 420 (e.g., a ceramic housing). As explained above, the receive resonator 404 is typically implanted within the body, while the transmit resonator 402 is typically external to the body. The transmit and receive resonators 402 and 404 may include one or more electronic components (not shown), such as field-effect transistors (FETs), series inductors, and/or other electronic components.

In this embodiment, the receive resonator 404 further includes a metal disk 450 on a side of the receive resonator 404 opposite the transmit resonator 402. The metal disk 450 may be fabricated from, for example, titanium. The metal disk 450 includes an exterior surface 452 and an interior surface 454 (i.e., that faces the second coil element 414).

In the embodiment of FIG. 4, the receive resonator 404 also includes a metal ring 460 that circumscribes the metal disk 450. The metal ring 460 may be fabricated from the same metal as the metal disk 450 (e.g., titanium). The metal ring 460 may be welded or otherwise coupled to the metal disk 450, and functions as an interface between the metal disk 450 and the second housing 420. Further, the metal ring 460 may be brazed to or otherwise coupled to the second housing 420.

The first core 410 of the transmit resonator 402 is a closed, or solid core. That is, a center 470 of the first core 410 is continuous and contains the same magnetic material as the rest of the first core (such as ferrite). Accordingly, the first core 410 generally has a disk-shape (as opposed to a ring-shape).

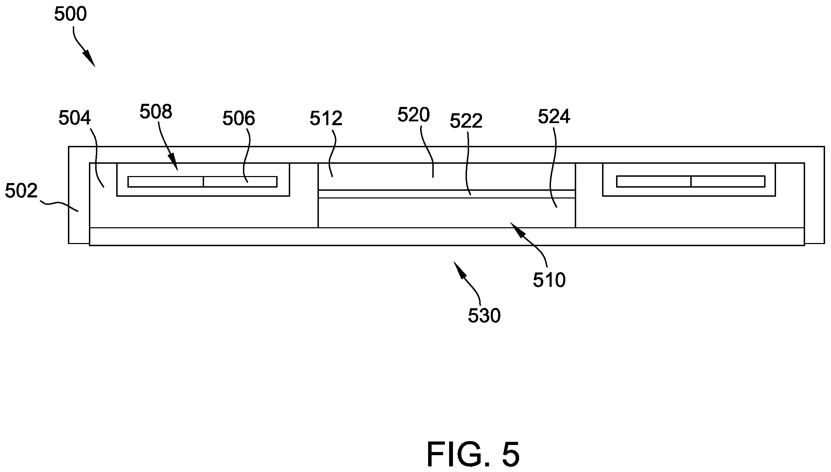

FIG. 5 is a cross-sectional view of an alternative embodiment of a transmit resonator 500 in accordance with the claimed invention that may be implemented with system 100 (shown in FIG. 1). The resonator 500 includes a housing 502, a core 504, and a coil element 506. The core 504 forms a u-shaped annular groove 508, and the coil element 506 is positioned in the annular groove 508.

As shown in FIG. 5, the core 504 is an open core. That is, the core 504 defines a central aperture 510 that does not contain a metallic or magnetic material. Instead, one or more layers 512 of non-magnetic, non-metallic materials are positioned in the central aperture 510. In the embodiment shown, the layers 512 include a first layer 520, a second layer 522, and a third layer 524. Alternatively, any suitable number of layers 520 may be included. Further, in the embodiment shown, the first, second, and third layers 520, 522, and 524 have different thicknesses. Alternatively, at least some of the first, second, and third layers 520, 522, and 524 may have the same thickness.

The first, second, and third layers 520, 522, and 524 may be made of the same material, or of different materials. Further, the materials used for the first, second, and third layers 520, 522, and 524 may include, for example, aluminum oxide, epoxy (e.g., EpoTek T7110), a printed circuit board (PCB) substrate material, etc. Those of skill in the art will appreciate that any suitable materials may be used. In some embodiments, at least one of the first, second, and third layers 520, 522, and 524 may include a layer of air.

Notably, the open core configuration of the core 504 shown in FIG. 5 results in lower emitted electromagnetic (EM) fields, as compared to a closed core, such as that shown in FIG. 4. Further, depending on the choice of the material(s) in the first, second, and third layers 520, 522, and 524, the overall temperature of the resonator 500 may also be lower, as compared to the transmit resonator shown in FIG. 4.

Magnetic cores (such as the first core 410 and the core 504) are typically used in wireless energy transfer systems to guide and focus the magnetic field created by a transmit resonator, such as the transmit resonator 102 and the resonator 500, toward a receive resonator, such as the receive resonator 104 (shown in FIG. 1). Such cores typically have a "U" shaped cross-section that forms a groove for the coil element. Thicknesses of the different sides of the "U" are typically non-uniform. For example, in at least some known cores, the side of the "U" closest to central axis of the coil is the thickest, and the magnetic field on this side is stronger as compared to other sides of the "U". The magnetic field is strong due to the magnetic field being concentrated into a relatively small diameter, thereby requiring more ferrite to avoid magnetic field saturation and losses associated with a high magnetic field per unit volume. For simplicity in design and manufacturing, and without negatively affecting the efficiency of the resonator, the thick side of the "U" is often connected to the coil's center. In other words, the thick side of the "U" is enabled to extend to the coil's center. This results in a solid, or closed core, such as the first core 410 shown in FIG. 4.

By connecting the "U" to the coil's center, the magnetic material in the coil's center creates a magnetic "shadow" immediately behind the core. This concept is discussed in U.S. Patent Application No. 17/341,818, filed June 8, 2021. This magnetic shadow protects electronic components in the resonator from magnetic fields.

In the resonator 500 (shown in FIG. 5), the absence of magnetic material in the central aperture 510 increases the electromagnetic (EM) field proximate a backside 530 of the resonator 530. However, only the near-field EM field increases; the far-field EM field generated by the resonator 500 actually decreases. As used herein, near-field generally refers to a distance from the coil element 506 that is less than or equal to an outer diameter of the core 504, and far-field generally refers to a distance from the coil element 506 that is greater than the outer diameter of the core 504 (including distance several times larger than the outer diameter of the core 504).

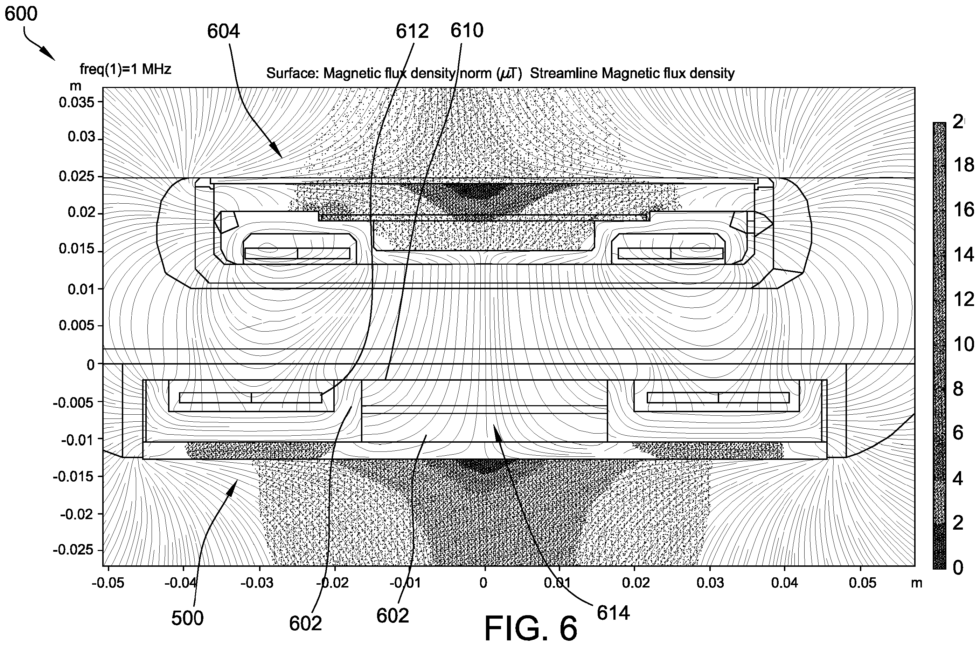

FIG. 6 is a diagram 600 illustrating magnetic field lines 602 through the resonator 500. For clarity, a receive resonator 604 is also shown. Notably, for the resonator 500 with an open core, the magnetic field lines 602 that would otherwise occupy a center of the core 504 shift position, such that those magnetic field lines 602 pass through a region between an inner radius 610 of the core 504 and a coil inner radius 612. This region includes ferrite material, which causes the magnetic field lines 602 to shift position because the magnetic pressure that keeps magnetic field lines 602 separated is reduce by the high magnetic permeability of the ferrite material. It is noted that not all of the magnetic lines 602 shift their position to pass through the ferrite material, as the magnetic permeability of the ferrite is not infinite. Accordingly, some of the magnetic field lines 602 remain between the inner radius of the core 504 and a center 614 of the resonator 500.

The shifting of the magnetic field lines 602 radially outward moves the magnetic field lines 602 closer to the coil element 506. As a result, the magnetic field lines 602 bend more sharply as they pass the coil element 506, and the trajectory of the magnetic field lines 602 will be pulled in closer to the resonator 500 and the receive resonator 604, reducing far-field EM emissions (e.g., by at least 5%). Notably, this phenomenon also increases the coupling between the resonator 500 and the receive resonator 604.

Additionally or alternatively, if the material(s) chosen for the layers 520, 522, and 524 (e.g., non-metallic and non-magnetic materials) have a thermal conductivity higher than ferrite, improved heat transport through the resonator 500 may be achieved, reducing the overall temperature of the resonator 500.

The embodiments described herein are directed to systems and methods for wireless power transfer. A resonator includes a housing, a magnetic core positioned within the housing, the magnetic core defining an annular groove and a central aperture, a coil element positioned within the annular groove, and at least one layer positioned within the central aperture, the at least one layer comprising a non-magnetic, non-metallic material.

Although the embodiments and examples disclosed herein have been described with reference to particular embodiments, it is to be understood that these embodiments and examples are merely illustrative of the principles and applications of the present disclosure. It is therefore to be understood that modifications can be made to the illustrative embodiments and examples and that other arrangements can be devised without departing from the scope of the present disclosure as defined by the claims.

This written description uses examples to disclose the disclosure, including the best mode, and also to enable any person skilled in the art to practice the disclosure, including making and using any devices or systems and performing any incorporated methods. The invention is defined by the now following claims.

## Claims

1. A resonator (500) for use in a transcutaneous energy transfer system (200) (TETS), the resonator comprising:

a housing (502);
a magnetic core (504) positioned within the housing, the magnetic core defining an annular groove (508); and
a coil element (506) positioned within the annular groove;
**characterized in that** the magnetic core further defines a central aperture (510), and **in that** the resonator (500) further comprises at least one layer (512) positioned within the central aperture (510), the at least one layer comprising a non-magnetic, non-metallic material.

2. The resonator (500) of claim 1, wherein the resonator is a transmit resonator.

3. The resonator (500) of claim 1, wherein the at least one layer (512) is fabricated from at least one of aluminum oxide, epoxy, and/or a printed circuit board (PCB) substrate material.

4. The resonator (500) of claim 1, wherein the at least one layer (512) comprises a plurality of layers.

5. The resonator (500) of claim 4, wherein the plurality of layers comprise a first layer (520) fabricated from a first material and a second layer (522) fabricated from a second, different material.

6. The resonator (500) of claim 4, wherein the plurality of layers have different thicknesses.

7. The resonator (500) of claim 4, wherein at least one layer of the plurality of layers is air.

8. A wireless power transfer system (200) comprising:

an implantable receive resonator (204); and
an external transmit resonator (202) comprising:

a housing (502);
a magnetic core (504) positioned within the housing, the magnetic core defining an annular groove (508); and
a coil element (506) positioned within the annular groove;
**characterized in that** the magnetic core further defines a central aperture (510), and **in that** the resonator (500) further comprises at least one layer (512) positioned within the central aperture (510), the at least one layer comprising a non-magnetic, non-metallic material.

9. The wireless power transfer system (200) of claim 8, wherein the at least one layer (512) is fabricated from at least one of aluminum oxide, epoxy, and/or a printed circuit board (PCB) substrate material.

10. The wireless power transfer system (200) of claim 8, wherein the at least one layer (512) comprises a plurality of layers.

11. A method of assembling a resonator (500) for use in a transcutaneous energy transfer system (TETS) (200), the method comprising:

positioning a magnetic core (504) within a housing (502), the magnetic core defining an annular groove (508); and
positioning a coil element (506) within the annular groove;
**characterized in that** the magnetic core further defines a central aperture (510), and **in that** the method further comprises positioning at least one layer (512) within the central aperture, the at least one layer comprising a non-magnetic, non-metallic material.

12. The method of claim 11, wherein the resonator (500) is a transmit resonator.

13. The method of claim 11, wherein positioning at least one layer (512) comprises positioning at least one layer fabricated from aluminum oxide.

14. The method of claim 11, wherein positioning at least one layer (512) comprises positioning at least one layer fabricated from epoxy and/or a printed circuit board (PCB) substrate material.

15. The method of claim 11, wherein positioning at least one layer (512) comprises positioning a plurality of layers.

**Patentansprüche**

1.  Resonator (500) zur Verwendung in einem transkutanen Energieübertragungssystem (200) (TETS), der Resonator umfassend:

    ein Gehäuse (502);
    einen magnetischen Kern (504), der innerhalb des Gehäuses angeordnet ist, wobei der magnetische Kern eine ringförmige Nut (508) definiert; und
    ein Spulenelement (506), das innerhalb der ringförmigen Nut angeordnet ist;
    **dadurch gekennzeichnet, dass** der magnetische Kern ferner eine Zentralöffnung (510) definiert und dass der Resonator (500) ferner mindestens eine Schicht (512) umfasst, die innerhalb der Zentralöffnung (510) angeordnet ist, wobei die mindestens eine Schicht ein nichtmagnetisches, nichtmetallisches Material umfasst.

2.  Resonator (500) nach Anspruch 1, wobei der Resonator ein Senderesonator ist.

3.  Resonator (500) nach Anspruch 1, wobei die mindestens eine Schicht (512) aus mindestens einem der folgenden Materialien hergestellt ist: Aluminiumoxid, Epoxid und/oder ein Leiterplatte-(PCB)-Substratmaterial.

4.  Resonator (500) nach Anspruch 1, wobei die mindestens eine Schicht (512) eine Mehrzahl von Schichten umfasst.

5.  Resonator (500) nach Anspruch 4, wobei die Mehrzahl von Schichten eine erste Schicht (520) umfasst, die aus einem ersten Material hergestellt ist, und eine zweite Schicht (522) umfasst, die aus einem zweiten, verschiedenen Material hergestellt ist.

6.  Resonator (500) nach Anspruch 4, wobei die Mehrzahl von Schichten verschiedene Dicken aufweist.

7.  Resonator (500) nach Anspruch 4, wobei mindestens eine Schicht der Mehrzahl von Schichten Luft ist.

8.  Drahtloses Energieübertragungssystem (200), umfassend:

    einen implantierbaren Empfangsresonator (204); und
    einen externen Senderesonator (202), umfassend:

    ein Gehäuse (502);
    einen magnetischen Kern (504), der innerhalb des Gehäuses angeordnet ist, wobei der magnetische Kern eine ringförmige Nut (508) definiert; und
    ein Spulenelement (506), das innerhalb der ringförmigen Nut angeordnet ist;
    **dadurch gekennzeichnet, dass** der magnetische Kern ferner eine Zentralöffnung (510) definiert und dass der Resonator (500) ferner mindestens eine Schicht (512) umfasst, die innerhalb der Zentralöffnung (510) angeordnet ist, wobei die mindestens eine Schicht ein nichtmagnetisches, nichtmetallisches Material umfasst.

9.  Drahtlose Energieübertragungssystem (200) nach Anspruch 8, wobei die mindestens eine Schicht (512) aus mindestens einem der folgenden Materialien hergestellt ist: Aluminiumoxid, Epoxid und/oder ein Leiterplatte-(PCB)-Substratmaterial.

10. Drahtlose Energieübertragungssystem (200) nach Anspruch 8, wobei die mindestens eine Schicht (512) eine Mehrzahl von Schichten umfasst.

11. Verfahren zum Zusammenbau eines Resonators (500) zur Verwendung in einem transkutanen Energieübertragungssystem (200) (TETS), das Verfahren umfassend:

    Positionieren eines magnetischen Kerns (504) innerhalb eines Gehäuses (502), wobei der magnetische Kern eine ringförmige Nut (508) definiert; und
    Positionieren eines Spulenelements (506) innerhalb der ringförmigen Nut;
    **dadurch gekennzeichnet, dass** der magnetische Kern ferner eine Zentralöffnung (510) definiert und dass das Verfahren ferner Positionieren mindestens einer Schicht (512) innerhalb der Zentralöffnung (510) umfasst, wobei die mindestens eine Schicht ein nichtmagnetisches, nichtmetallisches Material umfasst.

12. Verfahren nach Anspruch 11, wobei der Resonator (500) ein Senderesonator ist.

13. Verfahren nach Anspruch 11, wobei Positionieren mindestens einer Schicht (512) Positionieren mindestens einer Schicht umfasst, die aus Aluminiumoxid hergestellt ist.

14. Verfahren nach Anspruch 11, wobei Positionieren mindestens einer Schicht (512) Positionieren mindestens einer Schicht umfasst, die aus Epoxid und/oder einem Leiterplatte-(PCB)-Substratmaterial hergestellt ist.

15. Verfahren nach Anspruch 11, wobei Positionieren mindestens einer Schicht (512) Positionieren einer Mehrzahl von Schichten umfasst.

**Revendications**

1. Un résonateur (500) destiné à être utilisé dans un système (200) de transfert d'énergie transcutané (TETS), le résonateur comprenant :

   un boîtier (502) ;
   un noyau magnétique (504) positionné à l'intérieur du boîtier, le noyau magnétique définissant une rainure annulaire (508) ; et
   un élément bobine (506) positionné à l'intérieur de la rainure annulaire ;
   **caractérisé en ce que** le noyau magnétique définit en outre une ouverture centrale (510), et **en ce que** le résonateur (500) comprend en outre au moins une couche (512) positionnée à l'intérieur de l'ouverture centrale (510), ladite au moins une couche comprenant un matériau non magnétique et non métallique.

2. Le résonateur (500) selon la revendication 1, dans lequel le résonateur est un résonateur émetteur.

3. Le résonateur (500) selon la revendication 1, dans lequel ladite au moins une couche (512) est fabriquée à partir d'au moins un parmi l'oxyde d'aluminium, l'époxy et/ou un matériau de substrat de carte de circuit imprimé (PCB).

4. Le résonateur (500) selon la revendication 1, dans lequel ladite au moins une couche (512) comprend une pluralité de couches.

5. Le résonateur (500) selon la revendication 4, dans lequel la pluralité de couches comprend une première couche (520) fabriquée à partir d'un premier matériau et une seconde couche (522) fabriquée à partir d'un second matériau différent.

6. Le résonateur (500) selon la revendication 4, dans lequel la pluralité de couches présente des épaisseurs différentes.

7. Le résonateur (500) selon la revendication 4, dans lequel au moins une couche de la pluralité de couches est de l'air.

8. Un système (200) de transfert d'énergie sans fil comprenant :

   un résonateur récepteur implantable (204) ; et
   un résonateur émetteur externe (202) comprenant :

      un boîtier (502) ;
      un noyau magnétique (504) positionné à l'intérieur du boîtier, le noyau magnétique définissant une rainure annulaire (508) ; et
      un élément bobine (506) positionné à l'intérieur de la rainure annulaire ;
      **caractérisé en ce que** le noyau magnétique définit en outre une ouverture centrale (510), et **en ce que** le résonateur (500) comprend en outre au moins une couche (512) positionnée à l'intérieur de l'ouverture centrale (510), ladite au moins une couche comprenant un matériau non magnétique et non métallique.

9. Le système (200) de transfert d'énergie sans fil selon la revendication 8, dans lequel ladite au moins une couche (512) est fabriquée à partir d'au moins un parmi l'oxyde d'aluminium, l'époxy et/ou un matériau de substrat de carte de circuit imprimé (PCB).

**10.** Le système (200) de transfert d'énergie sans fil selon la revendication 8, dans lequel ladite au moins une couche (512) comprend une pluralité de couches.

**11.** Un procédé d'assemblage d'un résonateur (500) destiné à être utilisé dans un système (200) de transfert d'énergie transcutané (TETS), le procédé comprenant :

le positionnement d'un noyau magnétique (504) à l'intérieur d'un boîtier (502), le noyau magnétique définissant une rainure annulaire (508) ; et
le positionnement d'un élément bobine (506) à l'intérieur de la rainure annulaire ;
**caractérisé en ce que** le noyau magnétique définit en outre une ouverture centrale (510), et **en ce que** le procédé comprend en outre le positionnement d'au moins une couche (512) à l'intérieur de l'ouverture centrale, ladite au moins une couche comprenant un matériau non magnétique et non métallique.

**12.** Le procédé selon la revendication 11, dans lequel le résonateur (500) est un résonateur émetteur.

**13.** Le procédé selon la revendication 11, dans lequel le positionnement d'au moins une couche (512) comprend le positionnement d'au moins une couche fabriquée à partir d'oxyde d'aluminium.

**14.** Le procédé selon la revendication 11, dans lequel le positionnement d'au moins une couche (512) comprend le positionnement d'au moins une couche fabriquée à partir d'époxy et/ou d'un matériau de substrat de carte de circuit imprimé (PCB).

**15.** Le procédé selon la revendication 11, dans lequel le positionnement d'au moins une couche (512) comprend le positionnement d'une pluralité de couches.

FIG. 1

FIG. 2

FIG. 3

FIG. 4

EP 4 440 680 B1

FIG. 5

EP 4 440 680 B1

freq(1)=1 MHz    Surface: Magnetic flux density norm (μT)  Streamline Magnetic flux density

FIG. 6

EP 4 440 680 B1

**REFERENCES CITED IN THE DESCRIPTION**

**Patent documents cited in the description**

- WO 63284278 A
- US 2021271790 A

- US 34181821